# EUROPEAN PATENT SPECIFICATION

(11) **EP 1 904 139 B1**
(45) Date of publication and mention of the grant of the patent: **08.09.2010**
(21) Application number: 06766111.6
(22) Date of filing: 03.07.2006
(51) Int. Cl.: A61M 25/10, A61B 17/22

(54) **BALLOON CATHETER**
BALLONKATHETER
CATHETER A BALLONNET

(30) Priority: 05.07.2005 US 695868 P; 14.10.2005 US 726160 P
(43) Date of publication of application: 02.04.2008
(60) Divisional application: 10170623.2
(73) Proprietor: Angioslide Ltd., 46733 Herzliya (IL)
(72) Inventor: HIRSZOWICZ, Eran, 52236 Ramat-gan (IL); DUBI, Shay, Tel-aviv (IL)
(74) Representative: Modiano, Micaela Nadia
(86) International application number: PCT/IL2006/000770
(87) International publication number: WO 2007/004221

(56) References cited:
- EP-A- 0 359 489
- WO-A-84/01513
- GB-A- 2 054 385
- US-A- 6 039 721

## Description

### Field of the Invention

The present invention relates to a catheter system suitable for the retrieval of debris and other solid or liquid matter from body passages, and the removal of said matter from the body. More particularly, the invention relates to a catheter system comprising two or more concentrically-arranged conduits and an inflatable element connected therebetween, wherein said inflatable element is arranged such that it may entrap solid or liquid matter in an internal annular space.

### Background of the Invention

Catheters are used in various interventional procedures for delivering therapeutic means to a treated site (e.g., body organ or passageway such as blood vessels). In many cases, a catheter with a small distal inflatable balloon is guided to the treated site. Once the balloon is in place it is inflated by the operator for affixing it in place, for expanding a blocked vessel, for placing treatment means (e.g., stent) and/or for delivering surgical tools (e.g. knives, drills etc.) to a desired site. In addition, catheter systems have also been designed and used for retrieval of objects such as stents from body passageways.

Two basic types of catheter have been developed for intravascular use: other-the-wire (OTW) catheters and rapid-exchange catheters.

OTW catheter systems are characterized by the presence of a full-length guide wire, such that when the catheter is in its *in situ* working position, said guide wire passes through the entire length of a lumen formed in, or externally attached to, the catheter. OTW systems have several operational advantages which are related to the use of a full length guide wire, including good stiffness and pushability, features which are important when maneuvering balloon catheters along tortuous and/or partially occluded blood vessels.

US 6,039,721 describes a balloon catheter system comprising two concentrically-arranged conduits, with a balloon connected between the distal regions thereof. The catheter system permits both expansion/deflation of the balloon and alteration in the length of the balloon when *in situ*, such that the balloon may be moved between extended and intussuscepted conformations. The catheter system is constructed in order that it may be use for two main purposes: firstly, treatment (i.e. expansion) of different-length stenosed portions of blood vessels with a single balloon and secondly, the delivery of either stents or medication to intravascular lesions, wherein the stent or medication is contained within the distally-intussuscepted portion of the balloon. When used for multiple, differing-length lesion expansion, the balloon is inserted into blood vessel in a collapsed, shortened, intussuscepted conformation, and is advanced until it comes to rest in the region of the shortest lesion to be treated. The balloon is then inflated and the lesion treated (i.e. expanded). Following deflation of the balloon, the distal end of the catheter system is moved such that the balloon becomes positioned in the region of the next-shortest lesion to be treated. The effective length of the balloon is then increased by moving the inner conduit in relation to the proximal conduit, following which the balloon is again inflated and the lesion treated. In this way, a series of different length stenoses- in order from the shortest to the longest- may be treated using a single balloon. When used for stent delivery, the stent is pre-loaded into a proximal annular space formed as a result of balloon intussusception. The balloon is then moved to the desired site and the stent delivered by means of moving the inner conduit distally (in relation to the outer tube), thereby "unpeeling" the stent from the catheter.

WO 00/38776 discloses a dual-conduit balloon catheter system similar in basic design to that described above in relation to US 6,039,721. This catheter system is intended for use in a vibratory mode in order to break through total occlusions of the vascular lumen. In order to fulfill this aim, the outer conduit has a variable stiffness along its length, while the inner conduit. In addition, the inner conduit while being intrinsically relatively flexible is stiffened by the presence of axial tensioning wires. These conduit design features are used in order to permit optimal translation of vibratory movements of the proximal end of the inner conduit into corresponding vibration of the distal tip thereof.

Despite the large number of different balloon catheter systems currently available, a need still exists for a system that can efficiently and safely collect plaque debris and other particulate matter from the lumen of internal body passages such as pathologically-involved blood vessels.

The primary object of the present invention is, therefore, to provide a balloon catheter system capable of collecting samples and/or debris from the body treated site and reducing the risk of distal embolization of any material that may be dislodged during inflation of the balloon at the treated site.

Another aim is to provide such a system in which the balloon length may be substantially shortened during use without unduly increasing internal pressure.

A further aim is to provide such a system in which the catheter tubing is of a construction suitable for withstanding the forces generated during balloon folding and unfolding.

Yet another aim is to provide such a system in which the balloon is of a shape and constructions that permits both optimal debris entrapment and low-profile folding within the passages to be treated.

Other objects and advantages of the invention will become apparent as the description proceeds.

GB-A-2 054 385 discloses three different embodiments of a balloon catheter for use in dilating arterial or venous occlusions. The catheter comprises an external conduit, a balloon element having a mouth portion (attachment shoulder) peripherally secured to the distal end of the catheter and a body portion initially inverted within the catheter. The balloon element is evertable out of the catheter in response to the exertion of fluid pressure within the catheter for substantially anisotropic expansion out go of the catheter and through the occluded section of the vessel in advance of substantial lateral expansion and, upon eversion out of the catheter, being laterally expansible in response to the continued exertion of fluid pressure internally of the catheter to an outside diameter equal to or greater than the internal diameter of the vessel, and means to selectively impart internal pressure to the catheter. The proximal end of outer and inner tube is completely closed by a piston plunger which acts as means for the introduction of an expansion fluid into the balloon. Moreover, in the initial state of the catheter disclosed in GB-A-2 054 385, the balloon is inverted and stored inside (within) the outer conduit of the catheter.

### Summary of the Invention

The present invention is therefor primarily directed to a balloon catheter system is further disclosed in claim 1. It comprises at least one inner conduit and one outer conduit mutually disposed one inside the other, such that their longitudinal axes either coincide or are substantially parallel to each other, said inner tube being movably disposed along a longitudinal (i.e. distal-proximal) axis wherein an inflatable element such as a balloon is attached between the distal regions of said conduits, the distal end of the balloon being attached to the distal region of the inner conduit, and the proximal end of the balloon being attached to the distal region of said outer conduit. It is to be noted that the terms "distal" and "proximal" are defined in relation to the operator, i.e. the proximal direction is the direction that is in the direction of the operator, while the distal direction is the direction away from the operator, into the interior of the patient's body. The catheter system is constructed such that the longitudinal position of the inner conduit in relation to the outer conduit may be altered by means of moving the proximal end of the inner conduit (i.e. the end closest to the operator). In this way, the distal-proximal length of the outer surface of the balloon may be altered, such that the balloon may be caused to progressively move between an elongated, extended conformation and a shortened, terminally-intussuscepted conformation, wherein in the latter conformation, an openended inner lumen is created in the terminally-intussuscepted region of the balloon. In use, this inner lumen may be employed to entrap particulate debris, liquids and other objects and substances and safely remove same from the body passage in which the balloon catheter system is inserted. The inflatable element used in the presently-disclosed and described catheter system is of a shape that permits said element to meet the dual requirements of effective debris collection and low-profile delivery and retrieval. Thus, in one preferred embodiment the inflatable element is provided in the form of a balloon having, in its expanded state, a tapered shape with a rounded distal and/or proximal extremity. A further feature of the presently-disclosed system is the presence of means for preventing internal pressure changes that occur as a consequence of changing the length and conformation of the balloon.

Thus, in one aspect, the present invention provides a balloon catheter system comprising an outer conduit; an inner conduit, suitable for passage over a guide wire, disposed within the lumen of said outer conduit such that the longitudinal axes of said inner and outer conduits are substantially parallel, and positioned such that the distal tip of said inner conduit extends beyond the distal tip of said outer conduit, wherein said inner conduit is capable of being moved along its longitudinal axis in relation to said outer conduit; an angioplastic balloon whose proximal margin is attached to the outer surface of the distal tip of said outer conduit, and whose distal margin is attached to the outer surface of the portion of the inner conduit that extends beyond the distal tip of said outer conduit, and wherein the distal and/or proximal end portion(s) of said balloon are capable of intussusception upon proximal movement of said inner conduit in relation to said outer conduit; means for the introduction of an expansion fluid into the annular space formed between the inner surface of the outer conduit and the outer surface of the inner conduit and therefrom into the lumen of said balloon, and for the removal thereof, and means for preventing pressure changes within said annular space upon axial movement of said inner conduit in relation to said outer conduit.

In one preferred embodiment of the balloon catheter system defined hereinabove, said system is constructed such that the distal portion of the balloon is capable of intussusception upon proximal movement of the inner tube in relation to the outer tube.

In one preferred embodiment of the balloon catheter system of the present invention, the inner and outer conduits are characterized by their ability to withstand axially directed forces in the range of between 2 and 20 Newton without undergoing significant deformation. In the context of the present invention, the term "significant deformation" refers to changes in conduit length in excess of 5% of the total length of said conduit. While these conduits may be constructed of any suitable material capable of withstanding the aforementioned forces, in a preferred embodiment, the inner and outer conduits are constructed either from a biocompatible polymer (which in a preferred embodiment is selected from the group consisting of braided nylon thread and nylon thread that has undergone orientation treatment) or from flexible stainless steel tube.

In one preferred embodiment of the present invention, the balloon is characterized by having, in its inflated state, a pre-folding profile, i.e. it has shape which is capable of assisting and guiding the intussusception of the distal and/or proximal portion(s) thereof upon proximal movement of the inner conduit in relation to the outer conduit.

In one particularly preferred embodiment of the catheter system, the aforementioned balloon pre-folding profile is achieved by manufacturing the balloon such that it has (in its inflated state) a tapered shape with a rounded distal extremity.

In another preferred embodiment, the balloon has a tapered shape with a rounded proximal extremity.

Preferably, the balloon is constructed from Nylon, Pevax or mixtures thereof. It is to be recognized, however, that the balloon may also be constructed of any other suitable materials as are well known in the art, without deviating from the scope of the present invention as defined in the claims.

In one preferred embodiment, the aforementioned means for preventing pressure changes comprises a syringe-like structure positioned at the proximal end of the catheter system, wherein the barrel of said syringe-like structure is formed by an expanded portion of the outer conduit, and wherein the plunger of said structure co-axially surrounds the proximal end of the inner conduit, and is affixed thereto.

In another aspect, the present invention also provides a method for collecting debris from an interval passage of a mammalian subject comprising the steps of:
a) inserting a balloon catheter system as defined hereinabove over a guidewire into said internal passage, and advancing said catheter until the distal tip thereof has reached the site, at which it is desired to collect debris;
b) inflating the balloon with expansion fluid;
c) pulling the inner conduit of said balloon catheter in a proximal direction, such that the distal and/or proximal end(s) of said balloon intussuscept(s);
d) deflating the balloon, thereby forming a cavity into which debris is collected and entrapped; and
e) removing the balloon catheter from the internal passage of the subject, together with the entrapped debris.

In one preferred embodiment of the presently-disclosed method, the aforementioned internal passage is a vein or artery.

All the above and other characteristics and advantages of the present invention will be further understood from the following illustrative and non-limitative examples of preferred embodiments thereof.

### Brief Description of the Drawings

The present invention is illustrated by way of example in the figures of the accompanying drawings, in which like references indicate similar elements and in which:
- Fig. 1A schematically illustrates over-the-wire insertion of the balloon catheter of the invention;
- Fig. 1B shows a cross sectional side view of the balloon catheter of the invention;
- Fig. 1C schematically illustrates one embodiment of the syringe-like pressure-compensating device situated at the proximal end of the catheter system;
- Fig. 2 schematically illustrates the balloon catheter of the invention when inflated at a treatment site;
- Figs. 3 and 4 schematically illustrate debris collection carried out by the balloon catheter of the invention by folding the inflated balloon and deflating it thereafter; and
- Fig. 5 is a flowchart demonstrating the steps of an interventional procedure performed with the balloon catheter of the invention that may involve sample collection;
- Fig. 6 schematically illustrates the four balloon designs that were analyzed and compared in the finite element analysis study: a. Standard 20° tapering; b. 20° tapering with smooth round ending; c. Round ending; d. Round ending with initial retracting;
- Fig. 7 graphically depicts the displacement vs. retracting force for the four balloon shapes, compared at an inflation pressure of 6 atmospheres.
- Fig. 8 graphically depicts the maximum force generated in the catheter tubes following balloon folding, measured for different balloon inflation pressures;

### Detailed Description of Preferred Embodiments

The present invention is directed to a method and apparatus for removing objects (such as atheromatous plaque debris) or collecting samples from a body passageway such as a blood vessel. The presently-disclosed method and apparatus may also be used to expand a region of a body passageway (such as an atheromatous narrowing or occlusion of a blood vessel) in addition to removing debris or other matter or objects therefrom. In a preferred embodiment of the invention, a balloon catheter that is suitable for carrying out common interventional procedures is adapted to enable the expansion of a region of a body passageway and collection of objects or samples from the treated site utilizing a unique design of catheter and balloon.

In the following description, the terms "conduit" and "tube" are used interchangeably.

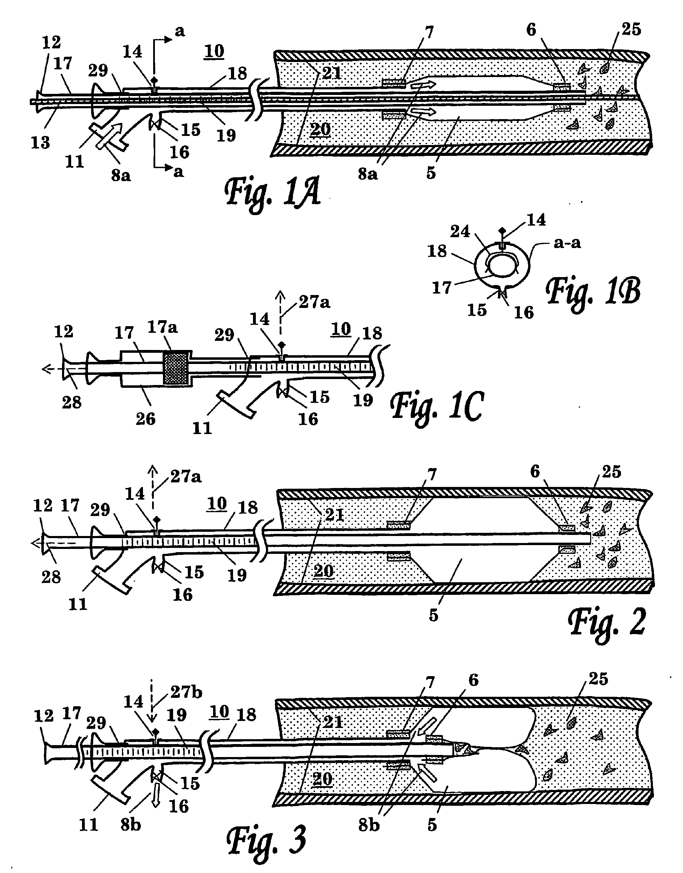

Referring to Fig. 1A which illustrates the insertion of the balloon catheter **10** of the invention to a treatment site, for example body passage **20.** As shown, balloon catheter **10** comprises an inner tube **17** slidably positioned inside outer tube **18.** The proximal (i.e., trailing) end of inner tube **17** comprises an entry port **12,** which extends outwardly through orifice **29** provided at the proximal end of outer tube **18.** Orifice **29** tightly fits around the outer surface of inner tube **17** without gripping it, thereby allowing proximal and distal movements of inner tube **17** while sealing the inner lumen of outer tube **18.** Graduated scale **19** may optionally be provided on the outer surface of inner tube **17.**

The proximal end of outer tube **18** further comprises a fluid port **11** for injecting/removing inflation fluids to/from inner lumen of outer tube **18,** an over-pressure valve outlet **15** for discharging inflation fluids whenever over-pressure conditions develop in the inner lumen of outer tube **18,** and an inner tube safety lock **14** adapted for gripping the outer surface of inner tube **17,** thereby preventing proximal-distal movements thereof relative to outer tube **18.**

Over-pressure valve outlet **15** may include an over-pressure valve **16** for sealing the opening of over-pressure valve outlet **15** and for discharging portions of inflating fluids therethrough whenever over-pressure conditions are reached in inner lumen of outer tube **18.** It should be realized however that such over-pressure conditions may be resolved by other means. For example, an inflatable member (not shown) may be attached to the opening of over-pressure valve outlet **15,** and in such an implementation over-pressure valve **16** may be eliminated. Moreover, outer tube **18,** or portions thereof, may be inflatable such that over-pressure conditions may be resolved by its expansion.

Inner tube safety lock **14** contacts the Outer surface of inner tube **17** via a tight orifice provided on the outer surface at the proximal end of outer tube **18.** As shown in the cross sectional view of Fig. 1B, a "U"-shaped gripping clip **24** may be attached to inner tube safety lock **14** for gripping inner tube **17** therewith by pushing inner tube safety lock **14** inwardly and fitting the arms of gripping clip **24** around the outer surface of inner tube **17.**

As seen in Fig. 1A distal (leading) end of inner tube **17** extends outwardly via the distal opening of outer tube **18,** into the body passage **20.** An inflatable member, for example non-compliant balloon **5,** is attached to the distal ends of outer tube **18** and inner tube **17.** Balloon **5** is preferably made from a flexible resilient sleeve having conical ends having gradually decreasing diameters towards the tips of the sleeve. Balloon **5** is attached at circumferential attachment point **7** to the outer surface near the distal tip of outer tube **18,** and at circumferential attachment point **6** to the outer surface near the distal tip of inner tube **17,** such that it seals the distal opening of outer tube **18.**

As mentioned hereinabove, in one preferred embodiment of the invention, the means for preventing pressure changes in the inflation fluid space comprises a syringe-like structure positioned at the proximal end of the catheter system, wherein the barrel of said syringe-like structure is formed by an expanded portion of the outer conduit, and wherein the plunger of said structure co-axially surrounds the proximal end of the inner conduit. Referring now to Fig. 1C, the mechanism in this preferred embodiment consists of a barrel portion **26** and plunger **17a** movably disposed therein and affixed to outer surface of inner tube **17.** Plunger **17a** seals the inflation lumen of balloon catheter **10,** such that proximal movements thereof, responsive to proximal movements of inner tube **17,** generates suction of inflation media into barrel portion **26.**

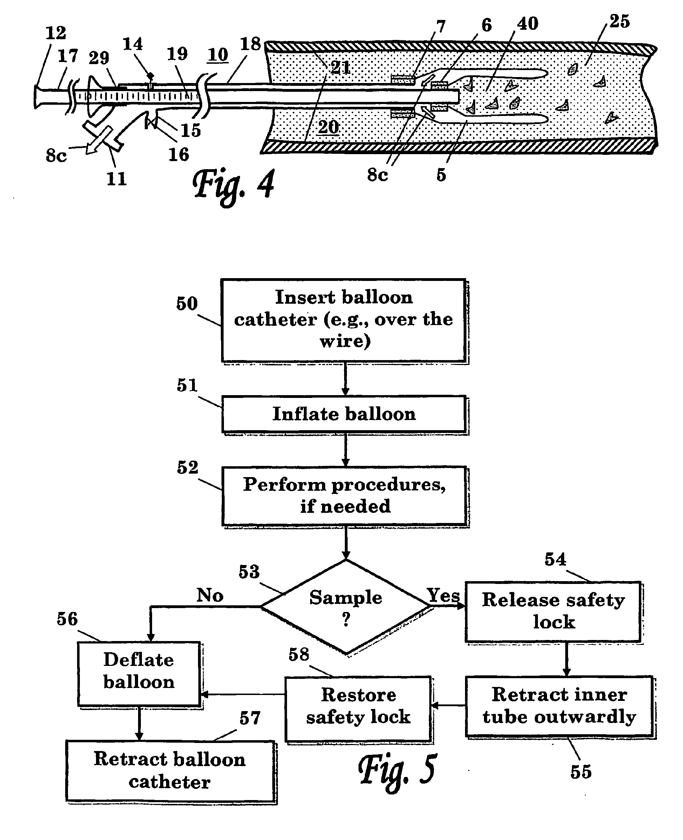

With reference to the flowchart of Fig. 5, demonstrating the steps of an interventional procedure performed with the balloon catheter of the invention. The procedure starts in step **50** wherein the balloon catheter **10** is guided to the treatment site (e.g., over the wire). Fig. 1A demonstrates over-the-wire insertion, wherein the insertion of balloon catheter **10** is performed over guide wire **13.** It should be clear, however, that the invention is not limited to one specific insertion method and that other appropriate and practicable insertion methods (e.g., using a guiding catheter) may also be used.

Next, in step **51,** the operator inflates balloon **5** by injecting inflation fluids via fluid port **11** and the inner lumen of outer tube **18,** as demonstrated by fluid inflation arrows **8a** in Fig. 1A. When carrying out procedures in body passage **20** as demonstrated in the Figs. 1-4 inflation fluids are preferably injected into balloon **5** such that its circumferential sides are expanded and pressed against the inner wall **21** of body passage **20,** as demonstrated in Fig. 2. The pressure inside balloon **5** in such conditions may be in general about 1-25 Atmospheres, preferably about 6 Atmospheres.

In this state in which the balloon catheter **10** is anchored, the inner lumen of inner tube **17** may now be utilized for operating in the treated site with different interventional tools (not shown), as may be required. Step **52** indicates the possibility of performing procedures if needed, however, some procedures (for example angioplasty) may be completed, or be near completion, once balloon **5** reaches its inflated state.

If it is determined in step **53** that a sample or other liquid or solid matter should be collected from the treatment site, for example fluids, secretions, and/or debris **25**, then in step **54** inner tube safety lock **14** is pulled thereby releasing its grip from inner tube **17,** as demonstrated by arrow **27a** in Fig. 2. In step **55** the inner tube **17** is retracted outwardly (proximally) by the operator as shown by arrow **28.** During retraction of inner tube **17** the distal tip of balloon **5** collapses and the outer surface portions are folded inwardly over the distal tip of inner tube **17** and thereafter over itself as further portions of the balloon collapse, as illustrated in Fig. 3.

Retraction of inner tube **17** and the resulting inward folding of balloon **5** shortens the overall length of inflated balloon **5** which actually reduces the volume of inflated balloon **5.** Consequently, the pressure exerted by the inflating fluids increases, resulting in a considerable pressure increase in balloon **5** and inner lumen of outer tube **18.** Whenever the pressure in balloon **5** and inner lumen of outer tube **18** reaches a certain set-point (e.g., 5-20 atmospheres) inflation fluids are discharged via over-pressure valve outlet **15,** as shown by arrows **8b** in Fig. 3, such that the pressure in balloon **5** and inner lumen of outer tube **18** remains within a predetermined pressure range (e.g., 5-20 atmospheres). During this step the operator can determine via graduated scale **19** the amount of length of inner tube **17** that has been retracted and in this way determine when to stop the retraction and restore immobilization (step **58)** of inner tube **17** by pushing down inner tube safety lock **14,** as indicated by arrow **27b.**

Next, in step **56,** balloon **5** is deflated by retracting inflation fluids via fluid port **11,** as indicated by arrows **8c** in Fig. 4. In result, the pressure inside balloon **5** and inner lumen of outer tube **18** is substantially decreased, and balloon **5** is deflated. The reduction in the volume of balloon **5** results in the formation of an inner lumen **40** defined by the outer surface of the folded balloon section, as shown in Fig. 4. In step **57** the operator retracts balloon catheter **10** proximally such that portion of fluid/secretion and debris **25** confined within inner lumen **40** are withdrawn with the balloon catheter **10** (not shown in the figures). The debris, objects or samples collected may be easily collected when the entire length of balloon catheter **10** is ejected from the body of the treated subject, by pushing the inner tube **17** distally and unfolding the folded portions of balloon **5,** thus restoring the deflated state of balloon **5** (shown in Fig. 1A).

In view of the axially-directed stretching and buckling forces exerted on the inner and outer tubes during elongation and shortening of the balloon, said tubes need to be constructed such that they are able to withstand axially-directed forces in the range of between 2 and 20 Newton without undergoing deformation. In order to achieve this aim, the conduits may be constructed of a braided material or of materials having a defined molecular orientation. The approximate maximum forces that the inner and outer tubes need to withstand (for two difference size ranges of balloon) are as follows:
- 2.5-4 mm balloons: the tubing should withstand up to 500g; polymer tubing made of nylon or pevax reinforced during the manufacturing process can be used.
- 4-5 mm (or larger) balloons: the tubing should withstand forces up to 2 kg. In this case it will be necessary to use a braided tube (polymer tube with metal mesh reinforcement).

Results for a representative study of the forces generated during balloon folding are presented in Example 2, hereinbelow.

Outer tube **18** is preferably made from a biocompatible polymer type of material, such as polyurethane or nylon or PET, and may be manufactured utilizing conventional methods, such as extrusion. The diameter of inner lumen of outer tube **18** is generally in the range of 0.5-2.0 mm (millimeters), preferably about 0.7 mm, and the diameter of fluid port **11** is generally in the range of 2-6 mm, preferably about 4 mm. The diameter of over-pressure valve outlet **15** is generally in the range of 1-6 mm, preferably about 4 mm, and the entire length of outer tube **18** is generally in the range of 100-2000 mm, preferably about 1400 mm.

Inner tube **17** is preferably made from a biocompatible polymer type of material, such as polyurethane or nylon or PET, and it may be manufactured utilizing conventional methods, such as extrusion. The diameter of inner lumen of inner tube **17** is generally in the range of 0.2-2.0 mm, preferably about 0.5 mm, and its entire length is generally in the range of 100-2000 mm, preferably about 1500 mm.

While the diameter of orifice **29** provided at the proximal tip of outer tube **18** should be adapted to provide appropriate sealing of inner lumen of outer tube **18** it should also close over the outer surface of inner tube **17** such that inner tube **17** may be displaced therethrough with relatively low frictional forces. For example, if the diameter of inner tube **17** is 0.7 mm, then the diameter of orifice **29** should be 1.0 mm.

Balloon **5** is preferably a non-compliant or semi-compliant balloon such as manufactured by Advanced Polymers (Salem, USA) and by Interface Associates (CA). It may be manufactured utilizing conventional methods known in the balloon catheter industry from a non-compliance type of material such as Pebax or Nylon (preferably Nylon 12). Its length is generally in the range of 10-60 mm, preferably about 20 mm. The body diameter can vary from 2.0 mm to 5 mm for coronary artery applications, and be significantly larger for use in larger blood vessels. Preferably, the balloon should have a burst pressure within the range of 12-20 atmospheres. The proximal and distal edges of balloon **5** are preferably adhered to the outer surfaces of outer tube **18** and inner tube **17** respectively, at circumferential attachment points **7** and **6** respectively, by utilizing a UV or thermobonding type of adhesive such as commonly used in the art.

The shape of balloon **5** has been found by the present inventors to be critical in order for said balloon to fulfill its intended functions in the presently-disclosed and claimed catheter system, namely:
i. to facilitate folding in such a way that the desired annular space is formed at the distal end of the intussuscepted balloon, by the application of the lowest possible retracting force;
ii. to present a low profile that will facilitate introduction and withdrawal of the deflated balloon into and out of the catheter system and body passage.

The materials and design of the balloon, especially the shape of the distal taper and the relationship between the distal and the proximal taper, thus allow the balloon to fold smoothly and with relatively low pulling forces. This also insures that the balloon will fold only its distal side.

It appears, from modeling studies performed by the inventors, that a tapered balloon with a smooth round ending folds best and has a relatively low retracting force, when compared to standard tapered balloon or a balloon with a round ending. In a particularly preferred embodiment, the balloon has a proximal taper cone shaped with a 15 - 17 degree angle, and a 15 degree round cone distal taper, having a radius of about 0.5 mm at the junction of the taper and the neck. The results of the aforementioned modeling studies are presented in Example 2, hereinbelow.

Inner tube safety lock **14** is preferably made from a biocompatible polymer such as Tecoflex; its length is generally in the range of 1-15 mm, preferably about 5 mm. If, for example, the cross-sectional diameter of inner tube safety lock **14** is about 2 mm, then the orifice provided on the outer surface of outer tube **18** through which inner tube safety lock **14** accesses inner lumen of outer tube **18** is preferably about 2.4 mm for providing suitable sealing of inner lumen of outer tube **18.**

### EXAMPLES

### Example 1

### Finite element analysis (FEA) of a debris-collecting balloon for use in the present invention

FEA is a computerized tool which was used to optimize the balloon design in order to improve its ability to fold in the desired way. The FE model describes an inflated balloon which its edge is retracted, resulting in folding of the balloon. The simulation was performed on different balloon designs and at varied inflation pressures, taking into account the mechanical properties of the balloon material, which was chosen to be nylon 12 or pebax.

### Assumptions:

i. The balloon is made of a homogenous and isotropic material.
ii. The balloon's shape is symmetrical around its longitudinal axis.
iii. The balloon's shape is symmetrical around its mid transverse axis.
iv. The folding results in flexural stresses in the balloon material. Thus the mechanical properties (Modulus and Poisson Ratio) of the substance when flexed are taken into account in the FE analyses.

### Methods:

a) The analyses were performed using a nonlinear Finite Elements Analysis (FEA) program MSC.MARC. This software allows assessment of the structural integrity and performance of parts undergoing large deformations as a result of thermal or structural load (www.mscsoftware.com).
b) The analyses were nonlinear, assuming large displacements and taking into account stiffness change due to geometry update and sequential forces.
c) The model was 2D axisymmetric.
d) The model consisted of about 1000 nodes and 1000 2D solid elements.
e) Constant pressure was applied from within the balloon on its walls, reflecting the inflation pressure. Simultaneously, gradually increased axial force was exerted to the edge of the balloon, results in its folding. The displacement of the balloon wall in the horizontal (longitudinal) axis was measured versus the applied force.
f) The longitudinal axis of the balloon was kept fixed, while the balloon walls were free to move/fold as a result of the axial load.
g) The balloon's specifications are listed in the following table:

| **Balloon Specifications** | | |
|---|---|---|
| Balloon length [mm] | | 20 |
| Balloon Outer Diameter [mm] | | 3 |
| Tube Outer Diameter [mm] | | 0.4 |
| Balloon Body Wall Thickness [µm] | | 10 |
| Neck Wall Thickness [µm] | | 50 |
| Tube Wall Thickness [µm] | | 100 |
| Tapering | | varying |
| Material | | PET (Polyethylene Terephthalate) |
| **Mechanical Properties** | | |
| | Flexural Modulus [Kg/mm²] | 100 |
| | Flexural Yield Strength [Kg/mm²] | 8.15 |
| | Poisson Ratio | 0.4 |

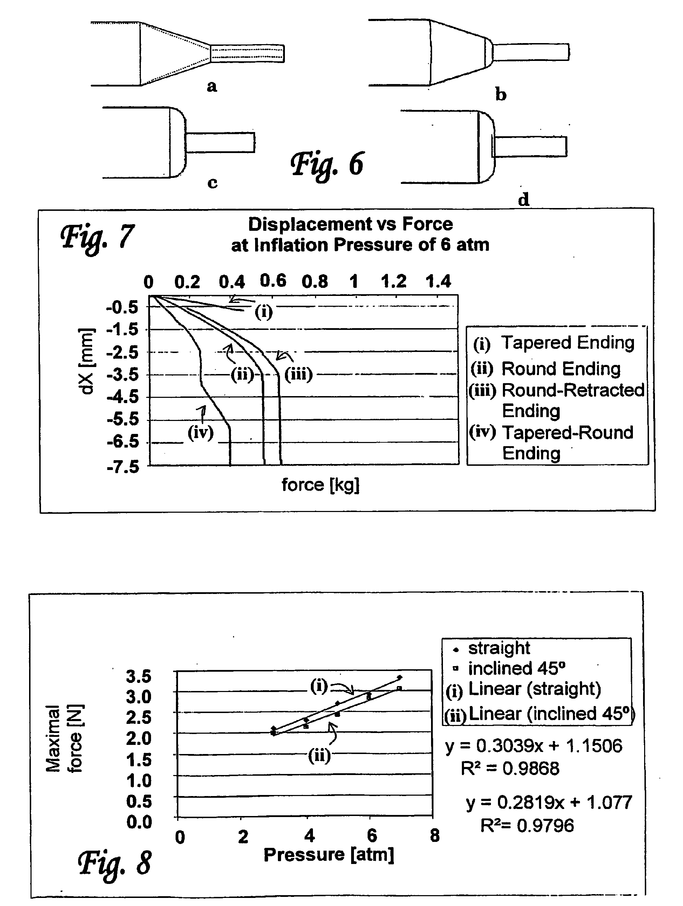

h) Four balloon designs were analyzed, wherein the differences reside in the design of their tapering (see Fig. 6):
Standard 20° tapering
20° tapering with smooth round ending
Round ending
Round ending with initial retracting portion.

i) The simulations were performed at five different inflation pressures: 1, 3, 6, 9 and 12 atmospheres.

### Results:

Fig. 7 shows the displacement vs. retracting force for the four balloon shapes at an inflation pressure of 6 atmospheres. Considering the maximal force required for collapse of the balloon, the Tapered-Round Ending Balloon required the lowest force, whereas the Round Ending Balloons need the greatest force to collapse. The Tapered Ending Balloon is somewhere between them. The slope of the Tapered Ending Balloon in the initial phase seems to be relatively moderate compared to the other balloon configurations. The moderate slope indicates higher stiffness. In other words, higher force is required to induce a given displacement. The slope of the Tapered-Round Ending Balloon is the steepest one, and suggests relatively high compliance to folding.

The balloon retracted shape vs. the original shape, at different inflation pressures was also studied (results not shown). The results demonstrated that the Tapered Ending Balloon is barely retracted, compared to the Round Ending Balloons which are retracted in a more smooth and continuous fashion. This is in spite of the higher force required to fold them.

### Conclusion:

From the above analyses it was concluded that the inflation pressure and the balloon geometry have an important role in determining of the required folding force and the folding style. It appears that a tapered balloon with a smooth round ending folds best and has a relatively low retracting force, when compared to standard tapered balloon or a balloon with a round ending.

### Example 2

### Determination of the force that is required in order to fold the balloon at different inflation pressure

### Equipment and materials:

3.0 mm Nylon 12 Vestamid L2101F Balloon (Interface Associates 316079-1)

Glass tube with inner diameter of 3 mm.
Guidant HI-TORQUE CROSS-IT 200XT 0.014" Guidewire. Hounsfield Test Equipment Model TX0927, 50-N load cell. This computer controlled testing machine enables determining tension, compression, shear, flexure and other mechanical and physical properties of materials. The machine provides selection of test speeds and direction of travel. It can measure the force and displacement values and can also graphically display the test.
Assouline Compressor type 1.5 HP.
Fluid dispensing system Model 1500XL.

### Procedure:

The balloon was inserted into a 3-mm glass tube, at straight position or inclined to 45°. A guidewire was inserted into the inner tube in order to stabilize the folding motion. The balloon was inflated using a compressor and the inflation pressure was controlled by a dispenser. The procedure was performed at pressures ranging between 3-7 atm, with increments of 1 atm. The balloon was folded using the Hounsfield Test machine, by pulling the inner tube at speed of 100 mm/min up to 20 mm, and then pushing back at the same speed until the balloon was completely unfolded.

Four tests were conducted at each pressure, to confirm that the results could be replicated.

### Results:

The maximal force required for folding the balloon at each pressure is presented in Fig. 8 The maximal force increases with the inflation pressure for both positions (straight and inclined) and ranges between 2-3.5 N (200-350 gr) with increments vary between 0.2-0.4 N (20-40 gr) per step of 1 atm in pressure. Higher inflation pressure requires greater force to fold the balloon. The relationship is approximately linear (R²=0.98). The maximal forces are slightly lower for the inclined position; however, repeated tests at the straight position revealed that the lesser forces result from the material fatigue. To support this assumption, visual examination of the balloon after 40 repeats showed that the balloon material lost its flexibility and looked crumpled.

The above examples and description have of course been provided only for the purpose of illustration, and are not intended to limit the invention in any way. As will be appreciated by the skilled person, the invention can be carried out in a great variety of ways, employing more than one technique from those described above, all without exceeding the scope of the invention.

## Claims

1. A balloon catheter (10) system comprising:
a) an outer conduit (18);
b) an inner conduit (17), suitable for passage over a guide wire (13), disposed within the lumen of said outer conduit (18) such that the longitudinal axes of said inner (17) and outer (18) conduits are substantially parallel, and positioned such that the distal tip of said inner conduit (17) extends beyond the distal tip of said outer conduit (18), wherein said inner conduit (17) is capable of being moved along its longitudinal axis in relation to said outer conduit (18);
c) an inflatable element (5) whose proximal margin is attached to the outer surface of the distal tip of said outer conduit (18), and whose distal margin is attached to the outer surface of the portion of the inner conduit (17) that extends beyond the distal tip of said outer conduit (18), and wherein the distal and/or proximal end portion(s) of said inflatable element (5) are capable of intussusception upon proximal movement of said inner conduit (17) in relation to said outer conduit (18);
d) means for the introduction of an expansion fluid into the annular space formed between the inner surface of the outer conduit (18) and the outer surface of the inner conduit (17) and therefrom into the lumen of said inflatable element (5), and for the removal thereof; and
e) an over-pressure valve (16) for preventing pressure changes within said annular space upon axial movement of said inner conduit (17) in relation to said outer conduit (18) and for discharging portions of inflating fluids therethrough whenever over-pressure conditions are reached in the inflatable element (5) and in the inner lumen of the outer tube (18).

2. The balloon catheter (10) system according to claim 1, wherein the distal portion of the inflatable element (5) is capable of intussusception upon proximal movement of the inner tube in relation to the outer tube.

3. The balloon catheter (10) system according to claim 1, wherein the inner and outer conduits (17, 18) are **characterized by** their ability to withstand axially directed forces in the range of between 2 and 20 Newton without undergoing significant deformation.

4. The balloon catheter (10) system according to claim 3, wherein the inner and outer conduits (17, 18) are constructed from a biocompatible polymer.

5. The balloon catheter (10) system according to claim 4, wherein the biocompatible polymer is selected from the group consisting of braided nylon thread and nylon 12 thread that has undergone orientation treatment.

6. The balloon catheter (10) system according to claim 3, wherein the inner and outer conduits (17, 18) are constructed from flexible stainless steel tube.

7. The balloon catheter (10) system according to claim 1, wherein the inflatable element (5) is **characterized by** having, in its inflated state, a shape which is capable of guiding the intussusception of the distal and/or proximal portion(s) thereof upon proximal movement of the inner conduit (17) in relation to the outer conduit (18).

8. The balloon catheter (10) system according to claim 7, wherein the inflatable element (5) is **characterized by** having, in its inflated state, a distal taper with a rounded distal extremity.

9. The balloon catheter (10) system according to claim 7, wherein the inflatable element (5) is **characterized by** having, in its inflated state, a proximal taper with a rounded proximal extremity.

10. The balloon catheter (10) system according to claim 1, wherein the inflatable element (5) is constructed from Nylon, Pevax or mixtures thereof.

11. The balloon catheter (10) system according to claim 1, further including a syringe-like structure positioned at the proximal end of the catheter system, wherein the barrel (26) of said syringe-like structure is formed by an expanded portion of the outer conduit (18), and wherein the plunger (17a) of said structure co-axially surrounds the proximal end of the inner conduit (17), and is affixed thereto.

12. The balloon catheter (10) system according to claim 1, wherein the proximal end of the outer tube (18) further comprises an over-pressure valve outlet (15) for discharging inflation fluids whenever over-pressure conditions develop in the inner lumen of outer tube (18).

13. The balloon catheter (10) system according to claim 12, wherein said over-pressure valve (16) is disposed within said over-pressure valve outlet (15).

14. The balloon catheter (10) system according to anyone of the preceding claims, wherein said catheter (10) system comprises an inner tube safety lock (14) adapted for gripping the outer surface of inner tube (17), thereby preventing proximale-distal movements thereof relative to the outer tube (18).

15. The balloon catheter (10) system according to anyone of the preceding claims, wherein said over-pressure conditions being reached in the inflatable element (5) and in the inner lumen of the outer tube (18) is a pressure comprised in the range from 5 to 20 atmospheres.

## Patentansprüche

1. Ein Ballonkatheter-(10)System, das Folgendes umfasst:
a) ein äußeres Rohr (18),
b) ein inneres Rohr (17), geeignet zum Durchlass über einen Führungsdraht (13), angeordnet innerhalb des Lumens des äußeren Rohrs (18), so dass die Längsachsen des inneren (17) und des äußeren (18) Rohrs im Wesentlichen parallel sind, und so positioniert, dass die distale Spitze des inneren Rohrs (17) sich über die distale Spitze des äußeren Rohrs (18) hinaus erstreckt, wobei das innere Rohr (17) entlang seiner Längsachse in Bezug zu dem äußeren Rohr (18) bewegt werden kann,
c) ein aufblasbares Element (5), dessen proximaler Rand an der äußeren Oberfläche der distalen Spitze des äußeren Rohrs (18) angebracht ist und dessen distaler Rand an der äußeren Oberfläche des Abschnitts des inneren Rohrs (17) angebracht ist, das sich über die distale Spitze des äußeren Rohrs (18) hinaus erstreckt, und wobei das/die distale(n) und/oder proximale(n) Endabschnitt(e) des aufblasbaren Elements (5) bei proximaler Bewegung des inneren Rohrs (17) in Bezug zu dem äußeren Rohr (18) zur Intussuszeption fähig sind,
d) Mittel zum Einführen eines Expansionsfluids in den ringförmigen Raum, der zwischen der inneren Oberfläche des äußeren Rohrs (18) und der äußeren Oberfläche des inneren Rohrs (17) gebildet ist, und von dort in das Lumen des aufblasbaren Elements (5), und zum Entfernen daraus, und
e) ein Überdruckventil (16) zum Verhindern von Druckänderungen innerhalb des ringförmigen Raums bei axialer Bewegung des inneren Rohrs (17) in Bezug zu dem äußeren Rohr (18) und zum Ablassen von Teilen auftreibender Fluide **dadurch** immer dann, wenn Überdruckbedingungen im aufblasbaren Element (5) und im inneren Lumen des äußeren Rohrs (18) erreicht sind.

2. Das Ballonkatheter-(10)System gemäß Anspruch 1, wobei der distale Abschnitt des aufblasbaren Elements (5) bei proximaler Bewegung des inneren Rohrs in Bezug zum äußeren Rohr zur Intussuszeption fähig ist.

3. Das Ballonkatheter-(10)System gemäß Anspruch 1, wobei das innere und das äußere Rohr (17, 18) durch ihre Fähigkeit **gekennzeichnet** sind, axial gerichteten Kräften im Bereich von zwischen 2 und 20 Newton zu widerstehen, ohne eine signifikante Verformung zu erfahren.

4. Das Ballonkatheter-(10)System gemäß Anspruch 3, wobei das innere und das äußere Rohr (17, 18) aus einem biokompatiblen Polymer hergestellt sind.

5. Das Ballonkatheter-(10)System gemäß Anspruch 4, wobei das biokompatible Polymer gewählt ist aus der Gruppe bestehend aus geflochtenem Nylonfaden und Nylon 12-Faden, der einer Orientierungsbehandlung unterzogen wurde.

6. Das Baiionkatheter-(10)System gemäß Anspruch 3, wobei das innere und das äußere Rohr (17, 18) aus flexibler rostfreier Stahlschlauchleitung hergestellt sind.

7. Das Ballonkatheter-(10)System gemäß Anspruch 1, wobei das aufblasbare Element (5) **dadurch gekennzeichnet ist, dass** es in seinem aufgeblasenen Zustand eine Form hat, die in der Lage ist, bei proximaler Bewegung des inneren Rohrs (17) in Bezug zum äußeren Rohr (18) die Intussuszeption der/des distalen und/oder proximalen Abschnitts/Abschnitte davon zu führen.

8. Das Ballonkatheter-(10)System gemäß Anspruch 7, wobei das aufblasbare Element (5) **dadurch gekennzeichnet ist, dass** es in seinem aufgeblasenen Zustand eine distale Verjüngung mit einem gerundeten distalen Ende hat.

9. Das Ballonkatheter-(10)System gemäß Anspruch 7, wobei das aufblasbare Element (5) **dadurch gekennzeichnet ist, dass** es in seinem aufgeblasenen Zustand eine proximale Verjüngung mit einem gerundeten proximalen Ende hat.

10. Das Ballonkatheter-(10)System gemäß Anspruch 1, wobei das aufblasbare Element (5) aus Nylon, Pervax oder Mischungen davon besteht.

11. Das Ballonkatheter-(10)System gemäß Anspruch 1, das weiter eine spritzenartige Struktur einschließt, die am proximalen Ende des Kathetersystems angebracht ist, wobei die Hülse (26) der spritzenartigen Struktur aus einem erweiterten Abschnitt des äußeren Rohrs (18) besteht und wobei der Druckkolben (17a) der Struktur das proximale Ende des inneren Rohrs (17) koaxial umgibt und daran befestigt ist.

12. Das Ballonkatheter-(10)System gemäß Anspruch 1, wobei das proximale Ende des äußeren Rohrs (18) weiter eine Überdruckventilöffnung (15) zum Ablassen von Aufblasfluiden immer dann, wenn sich Überdruckbedingungen im inneren Lumen des äußeren Rohrs (18) entwickeln, umfasst.

13. Das Ballonkatheter-(10)System gemäß Anspruch 12, wobei das Überdruckventil (16) in der Überdruckventilöffnung (15) angebracht ist.

14. Das Ballonkatheter-(10) System gemäß einem beliebigen der obigen Ansprüche, wobei das Katheter-(10)System einen Innenrohr-Sicherheitsriegel (14) umfasst, der ausgebildet ist, um die äußere Oberfläche des inneren Rohrs (17) zu greifen und so proximal-distale Bewegungen desselben in Bezug zum äußeren Rohr (18) zu verhindern.

15. Das Ballonkatheter-(10)System gemäß einem beliebigen der obigen Ansprüche, wobei die Überdruckbedingungen, die im aufblasbaren Element (5) und im inneren Lumen des äußeren Rohrs (18) erreicht werden, ein Druck im Bereich von 5 bis 20 Atmosphären sind.

## Revendications

1. Cathéter à ballonnet (10), comprenant :
a) un conduit extérieur (18) ;
b) un conduit intérieur (17), convenant pour le passage sur un fil de guidage (13), disposé à l'intérieur du lumen dudit conduit extérieur (18), de manière que les axes longitudinaux desdits conduits intérieur (17) et extérieur (18) soient sensiblement parallèles, et positionné de manière que le bout distal dudit conduit intérieur (17) s'étende au-delà du bout distal dudit conduit extérieur (18), dans lequel ledit conduit intérieur (17) peut être déplacé le long de son axe longitudinal par rapport audit conduit extérieur (18) ;
c) un élément gonflable (5), dont le bord proximal est attaché à la surface extérieure du bout distal dudit conduit extérieur (18), et dont le bord distal est attaché à la surface extérieure de la partie du conduit intérieur (17) s'étendant au-delà du bout distal dudit conduit extérieur (18), et dans lequel la ou les partie(s) d'extrémité distale et/ou proximale dudit élément gonflable (5) peuvent effectuer une intussusception lors du déplacement proximal dudit conduit intérieur (17) par rapport audit conduit extérieur (18) ;
d) des moyens pour l'introduction d'un fluide de dilatation dans l'espace annulaire formé entre la surface intérieure du conduit extérieur (18) et la surface extérieure du conduit intérieur (17) et, à partir de là, dans le lumen dudit élément gonflable (5), et pour son enlèvement ; et
e) une soupape de surpression (16), pour empêcher que des fluctuations de pression se produisent à l'intérieur de l'espace annulaire lors du déplacement axial dudit conduit intérieur (17) par rapport audit conduit extérieur (18), et pour décharger à travers celle-ci des parties des fluides de gonflage, si des conditions de surpression étaient atteintes dans l'élément gonflable (5) et dans le lumen intérieur du tube extérieur (18).

2. Cathéter à ballonnet (10) selon la revendication 1, dans lequel la partie distale de l'élément gonflable (5) peut effectuer une intussusception lors du déplacement proximal du tube intérieur par rapport au tube extérieur.

3. Cathéter à ballonnet (10) selon la revendication 1, dans lequel les conduits intérieur et extérieur (17, 18) sont **caractérisés par** leur aptitude à résister à des forces orientées axialement, d'une valeur dans la fourchette comprise entre 2 et 20 Newton, sans subir de déformation significative.

4. Cathéter à ballonnet (10) selon la revendication 3, dans lequel les conduits intérieur et extérieur (17, 18) sont construits en un polymère biocompatible.

5. Cathéter à ballonnet (10) selon la revendication 4, dans lequel le polymère biocompatible est sélectionné dans le groupe constitué de fil de nylon tressé et de fil de nylon 12 ayant subi un traitement d'orientation.

6. Cathéter à ballonnet (10) selon la revendication 3, dans lequel les conduits intérieur et extérieur (17, 18) sont constitués de tube en acier inoxydable flexible.

7. Cathéter à ballonnet (10) selon la revendication 1, dans lequel l'élément gonflable (5) est **caractérisé par** le fait d'avoir, à soin état gonflé, une forme permettant de guider l'intussusception de la ou des partie(s) distale et/ou proximale de celui-ci, lors du déplacement proximal du conduit intérieur (17) par rapport au conduit extérieur (18).

8. Cathéter à ballonnet (10) selon la revendication 7, dans lequel l'élément gonflable (5) est **caractérisé par** le fait d'avoir, à son état gonflé, un effilement distal, avec une extrémité distale arrondie.

9. Cathéter à ballonnet (10) selon la revendication 7, dans lequel l'élément gonflable (5) est **caractérisé par** le fait d'avoir, à son état gonflé, un effilement proximal, avec une extrémité proximale arrondie.

10. Cathéter à ballonnet (10) selon la revendication 1, dans lequel l'élément gonflable (5) est composé de Nylon, Pevax ou de mélanges de ceux-ci.

11. Cathéter à ballonnet (10) selon la revendication 1, comprenant en outre une structure analogue à une seringue, positionnée à l'extrémité proximale du système à cathéter, dans lequel le corps (26) de ladite structure analogue à une seringue est formé par une partie dilatée du conduit extérieur (18), et dans lequel le piston (17a) de ladite structure entoure coaxialement l'extrémité proximale du conduit intérieur (17), et est fixé à celui-ci.

12. Cathéter à ballonnet (10) selon la revendication 1, dans lequel l'extrémité proximale du tube extérieur (18) comprend en outre une sortie de soupape de surpression (15), pour décharger des fluides de gonflage, si des conditions de surpression se développent dans le lumen intérieur du tube extérieur (18).

13. Cathéter à ballonnet (10) selon la revendication 12, dans lequel ladite soupape de surpression (16) est disposée à l'intérieur de ladite sortie de soupape de surpression (15).

14. Cathéter à ballonnet (10) selon l'une quelconque des revendications précédentes, dans lequel ledit système à cathéter (10) comprend un verrou de sécurité de tube intérieur (14), adapté pour saisir la surface extérieure du tube intérieur (17), de manière à empêcher tout déplacement proximal-distal de celui-ci par rapport au tube extérieur (18).

15. Cathéter à ballonnet (10) selon l'une quelconque des revendications précédentes, dans lequel lesdites conditions de surpression atteintes dans l'élément gonflable (5) et dans le lumen intérieur du tube extérieur (18) sont une pression dans la fourchette comprise entre 5 et 20 atmosphères.
